# EUROPÄISCHE PATENTSCHRIFT

(11) **EP 0 406 455 B1**
(45) Veröffentlichungstag und Bekanntmachung des Hinweises auf die Patenterteilung: **21.09.1994**
(21) Anmeldenummer: 89112104.8
(22) Anmeldetag: 03.07.1989
(51) Int. Cl.: A61B 6/00, A61B 6/04

(54) **Röntgendiagnostikgerät für Mammographieaufnahmen**
X-ray diagnostic apparatus for mammographies
Appareil diagnostique à rayons X pour les mammographies

(43) Veröffentlichungstag der Anmeldung: 09.01.1991
(73) Patentinhaber: SIEMENS AKTIENGESELLSCHAFT, 80333 München (DE)
(72) Erfinder: Saffer, Edmund, D-8551 Eggolsheim (DE)

(56) Entgegenhaltungen:
- EP-A- 0 145 893
- DE-A- 3 437 203
- US-A- 4 212 306

## Beschreibung

Die Erfindung betrifft ein Röntgendiagnostikgerät für Mammographieaufnahmen mit einer Auflageplatte für ein Aufnahmeobjekt und mit einer senkrecht zur Auflageplatte über Halterungsmittel verstellbaren Kompressionsplatte.

Ein Röntgendiagnostikgerät der oben genannten Art ist beispielsweise durch das DE-GM 83 32 063 bekannt geworden. Bei diesem Gerät sind die Auflageebene einer Auflageplatte und die Kompressionsebene einer Kompressionsplatte parallel zueinander ausgerichtet. Zur Anfertigung einer Mammographieaufnahme wird die weibliche Brust auf die Auflageplatte aufgelegt und mittels der Kompressionsplatte, die in Richtung zur Auflageebene verstellt wird, komprimiert. Durch die immer parallel zueinander ausgerichtete Kompressions- und Auflageebene kann Brustgewebe beim Komprimieren in Richtung zu den Rippen verdrängt werden, was dazu führen kann, daß ein Teil des Brustgewebes nicht mehr auf dem Röntgenfilm darstellbar ist, weil es im Röntgenschatten der Rippen liegt. Außerdem kann Brustgewebe an den Rippen verdichtet werden, wodurch die Diagnoseerstellung anhand einer so angefertigten Aufnahme erschwert ist.

Aus der vorangemeldeten und nachveröffentlichten WO 89/11248 ist eine Kompressionsvorrichtung zum Komprimieren des Brustgewebes bekannt, wobei die Kompressionsfläche einer Kompressionsplatte und die Auflageebene einer Auflageplatte einen spitzen Winkel zur Patientenseite hin einnehmen.

Aufgabe der Erfindung ist es, ein Gerät der eingangs genannten Art so auszuführen, daß es die gleichmäßige Komprimierung des Brustgewebes ohne eine Verdrängung des Brustgewebes in Richtung zu den Rippen ermöglicht.

Die Aufgabe wird erfindungsgemäß durch die Merkmale des Patentanspruches 1 gelöst.

Vorteil der Erfindung ist, daß das Brustgewebe durch die schräg zur Auflageebene ausgerichtete Kompressionsfläche im Bereich der Rippen beginnend komprimiert wird, so daß sich das Brustgewebe in gewünschter Weise in Richtung von den Rippen weg verschiebt. Damit kann das gesamte Brustgewebe auf dem Röntgenfilm dargestellt werden, Gewebeverdichtungen an den Rippen werden weitgehend vermieden. Durch die bei Kompression verstellbare Kompressionsfläche ist sichergestellt, daß das Brustgewebe bei ausreichender Kompression gleichmäßig komprimiert ist. Es können also gut diagnostizierbare Mammographieaufnahmen angefertigt werden.

Weitere Vorteile und Einzelheiten der Erfindung ergeben sich aus der nachfolgenden Beschreibung von Ausführungsbeispielen anhand der Zeichnungen in Verbindung mit den Unteransprüchen.
- Figur 1: zeigt ein Röntgendiagnostikgerät mit einer Vorrichtung nach der Erfindung und
- Figur 2: ein weiteres Ausführungsbeispiel einer Vorrichtung nach der Erfindung.

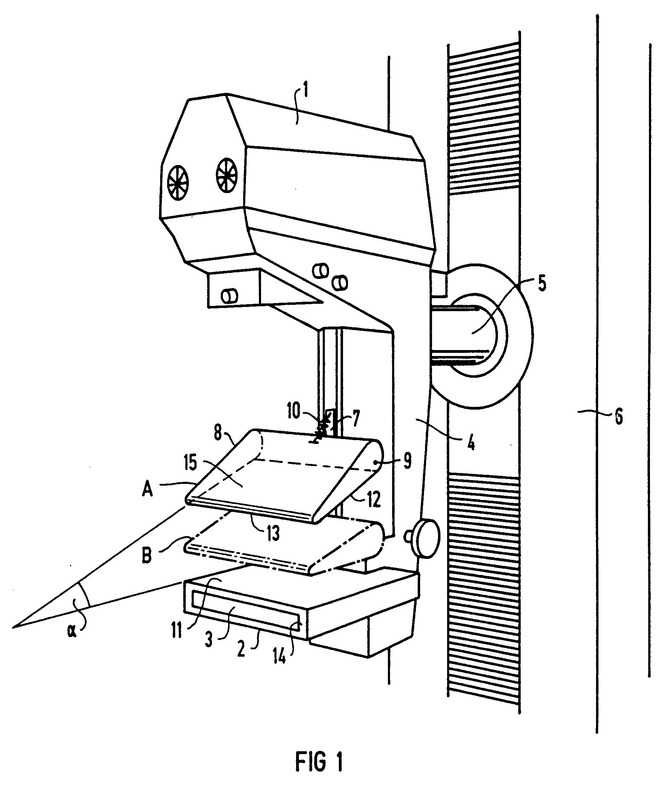

In der Figur 1 sind ein Gehäuse 1 für eine Röntgenröhre und eine Auflageplatte 2 zur Anfertigung üblicher Mammographien dargestellt. In die Auflageplatte 2 ist eine Röntgenfilmkassette 3 einschiebbar. Das Gehäuse 1 und die Auflageplatte 2 sind über einen Halter 4 miteinander verbunden, der eine horizontale Achse 5 aufweist, mit der er an einem Stativ 6 höhenverstellbar gelagert ist. Die Einheit 1, 2 ist ferner um die Achse 5 drehbar.

Im Halter 4 ist ein motorisch verstellbarer Kompressionswagen 7 vorgesehen, an dem eine Kompressionsplatte 8 höhenverstellbar gelagert ist. Sie ist um eine horizontale Achse 9 gegen die Kraft eines Federelementes 10 schwenkbar, das beispielsweise als Druck-Spiralfeder mit einem Ende an der dem Halter 4 nahen Seite der Kompressionsplatte 8 und mit dem anderen Ende am Kompressionswagen 7 befestigt ist. Die Figur 1 zeigt, daß die Kompressionsplatte 8 in der mit A gekennzeichneten Position schräg nach vorne geneigt ist, so daß die Auflageebene 11 der Auflageplatte 2 und die Kompressionsfläche 12 der Kompressionsplatte 8 einen spitzen Winkel Alpha zueinander einnehmen. Der Winkel Alpha kann beispielsweise durch die Länge des Federelementes 10 eingestellt werden. Die Figur 1 zeigt, daß sich der Raum zwischen der Auflageebene 11 und der Kompressionsfläche 12 zur patientennahen Seite, d.h. den Stirnseiten 13 und 14 der Kompressionsplatte 8 bzw. der Auflageplatte 2 hin verjüngt. In einer nicht gezeigten Ausführungsform der Erfindung kann die Kompressionsvorrichtung aus Kompressionsplatte 8 und Auflageplatte 2 auch um 90° um eine zum Halter 4 parallele Achse gedreht werden, so daß sich der Raum zwischen der Kompressionsfläche 12 und der Auflageebene 11 zu einer Seite 2 des Röntgendiagnostikgerätes hin verjüngt. So kann eine zu untersuchende Person zur Anfertigung einer Mammographieaufnahme auch seitlich neben dem Röntgendiagnostikgerät stehen, was bei bestimmten Untersuchungen von Vorteil ist.

Nachdem die weibliche Brust auf der Auflageebene 11 zur Anfertigung einer Mammographieaufnahme aufgelegt ist, wird zur Kompression die Kompressionsplatte 8 über den Kompressionswagen 7 in Richtung zur Auflageplatte 2 verstellt. Hierbei ist darauf zu achten, daß die vordere Stirnseite 13 der Kompressionsplatte 8 und die vordere Stirnseite 14 der Auflageplatte 2 in Kontakt zur Brustwand der zu untersuchenden Person stehen. Die Stirnseite 13 ist abgerundet, wobei die Kompressionsfläche 12 und die Oberseite 15 der Kompressionsplatte 8 keilförmig zur Stirnseite 13 ausgerichtet sind. Dadurch ist die Oberfläche im Bereich der Stirnseite 13 gering, so daß diese beim Anpressen der Brustwand an die Kompressionsplatte 8 sehr nahe an den Rippenbogen herangeführt werden kann. Beim Verstellen der Kompressionsplatte 8 in Richtung zur Auflageplatte 2 wird durch die Schrägstellung der Kompressionsfläche 12 zuerst der brustwandnahe Bereich des Brustgewebes komprimiert. Bei weiterer Kompression richtet sich die Kompressionsfläche 12 gegen die Kraft des Federelementes 10, dessen Federkraft einstellbar ist, allmählich parallel zur Auflageebene 11 aus, wobei das Brustgewebe in Richtung vom Rippenbogen weg verdrängt wird. Hierdurch ist sichergestellt, daß das gesamte Brustgewebe ohne Gewebeverdichtungen an den Rippen auf dem Röntgenfilm darstellbar ist. Bei ausreichender Kompression sind die Kompressionsfläche 12 und die Auflageebene 11 parallel zueinander ausgerichtet, wodurch das Brustgewebe möglichst gleichmäßig komprimiert ist und optimale Aufnahmeergebnisse erhalten werden. Zu Beginn der Kompression ist ausreichend Raum für die Hände des Untersuchers zum Einrichten der Mamma vorhanden.

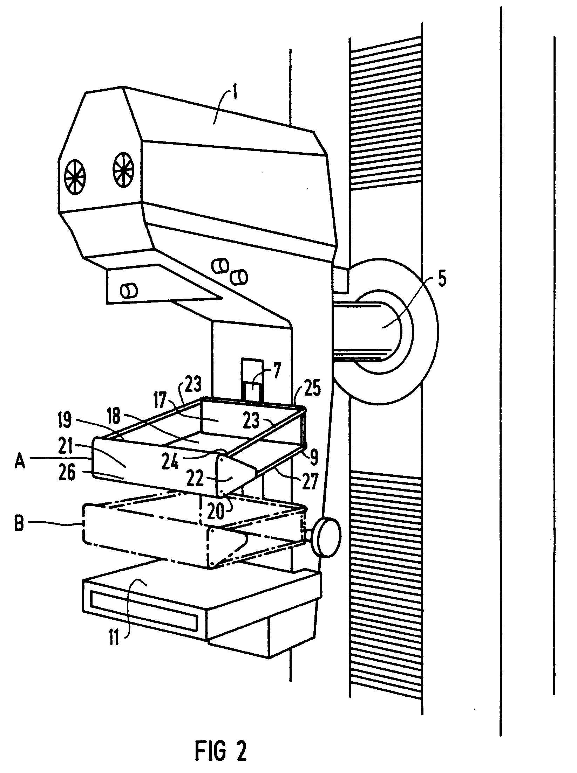

Die Figur 2 zeigt eine weitere erfindungsgemäß ausgestaltete Röntgendiagnostikanlage. Elemente, die bereits in der Fig. 1 gekennzeichnet wurden, erhalten dieselben Bezugszeichen. Eine Platte 17 ist am Kompressionswagen 7 befestigt und somit höhenverstellbar. Am unteren Ende der Platte 17 ist eine Kompressionsplatte 18 um die Achse 9 schwenkbar gelagert. Auch hier ist ein nicht gezeigtes Federelement vorgesehen, durch das die Kompressionsplatte 18 analog der Figur 1 nach vorne geneigt wird. An der patientennahen Stirnseite 26 der Kompressionsplatte 18 ist ein weiteres Abweiselement 19 angeordnet, das um eine zur Achse 9 parallele Achse 20 schwenkbar ist. Die Figur zeigt, daß das Abweiselement 19 eine Andruckfläche 21 mit zwei seitlich um 90° zur Andruckfläche 21 abgewinkelte, beispielsweise dreieckförmige Flächen 22 und eine Anlenkung 23 aufweist. An der patientennahen Stirnseite 26 der Kompressionsplatte 18 ist das Abweiselement 19 durch ein Lager gehalten, das beispielsweise als Kunststoffscharnier von einem Zapfen der dreieckförmigen Fläche 22, der in eine Bohrung der Kompressionsplatte 18 greift, gebildet ist. Durch diese Lagerung ist die Andruckfläche 21 um die Achse 20 schwenkbar. Vorteilhafterweise ist das Kunststoffscharnier so ausgebildet, daß beim Verschwenken der Andruckfläche 21 keine Verletzungsgefahr für die zu untersuchende Person besteht. Die Anlenkung 23 ist mit einem Ende 24 am oberen Ende der dreieckförmigen Flächen 22 und mit dem anderen Ende 25 am oberen Ende der Platte 17 gelagert. Die Anlenkung 23, die Kompressionsplatte 18, die Platte 17 sowie das Abweiselement 19 bilden somit eine Parallelogrammanordnung, durch welche die Verstellung der Kompressionsfläche 27 und der Andruckfläche 21 erfolgt. Durch Abstimmung der Lagerpunkte an der Platte 17 und an der dreieckförmigen Fläche 22 und Auslegung der Anlenkung 23 ist die Andruckfläche 21 in der mit A gekennzeichneten Position schräg zur Kompressionsfläche 27 der Kompressionsplatte 18 ausgerichtet. Bei Kompression der weiblichen Brust, d.h. bei Verstellung der Kompressionsvorrichtung aus Kompressionsplatte 18 und Abweiselement 19 in die mit B gekennzeichnete Position, richtet sich die Kompressionsfläche 27 der Kompressionsplatte 18 gegen die Kraft des Federelementes analog zur Anordnung nach Fig. 1 parallel zur Auflageebene 11 aus. Die Andruckfläche 21 wird dabei durch obengenannte Abstimmung um die Achse 20 verschwenkt, wobei der Winkel, den die Andruckfläche 21 zur Kompressionsfläche 27 einnimmt, vergrößert wird, bis sie schließlich annähernd senkrecht, vorzugsweise mindestens senkrecht, zur Kompressionsfläche 27 ausgerichtet ist. Hierdurch wird etwaiges Fettgewebe nach oben und damit aus dem Strahlengang verdrängt, so daß das Brustgewebe vollständig auf dem Röntgenfilm dargestellt werden kann.

Das Abweiselement 19 und die Kompressionsplatte 18 bilden im Bereich der patientennahen Stirnseite 26 eine abgerundete Fläche, so daß die Flächenpressung beim Andrücken der Brustwand an die Kompressionsvorrichtung aus Kompressionsplatte 18 und Abweiselement 19 verringert ist, wodurch Druckstellen und damit Schmerzen vermieden werden.

## Patentansprüche

1. Röntgendiagnostikgerät für Mammographieaufnahmen mit einer Auflageplatte (2) für ein Aufnahmeobjekt und mit einer senkrecht zur Auflageplatte (2) über Halterungsmittel (4, 7, 9) verstellbaren Kompressionsplatte (8, 18),
wobei die Kompressionsfläche (12, 27) der Kompressionsplatte (8, 18) und die Auflageebene (11) der Auflageplatte (2) in einer Position A einen spitzen Winkel zueinander einnehmen, so daß sich der Raum zwischen der Auflageebene (11) und der Kompressionsfläche (12, 27) zur Patientenseite hin verjüngt und
wobei die Stirnseite (13, 26) der Kompressionsplatte (8, 18) abgerundet ist.

2. Röntgendiagnostikgerät nach Anspruch 1,
**dadurch gekennzeichnet**,
daß die Kompressionsplatte (8, 18) über die Halterungsmittel (7, 9) derart gelagert ist, daß sich der Winkel, den die Kompressionsfläche (12, 27) zur Auflageebene (11) einnimmt, bei Kompression des Aufnahmeobjektes reduziert.

3. Röntgendiagnostikgerät nach Anspruch 1 oder 2,
**dadurch gekennzeichnet**,
daß die Auflageebene (11) senkrecht zu einem Halter (4) ausgerichtet ist und daß der Winkel, den die Auflageebene (11) und die Kompressionsfläche (12, 27) zueinander einnehmen, bei Kompression des Aufnahmeobjektes gegen die Kraft eines Federelementes (10) verringert wird.

4. Röntgendiagnostikgerät nach einem der Ansprüche 1 bis 3,
**dadurch gekennzeichnet**,
daß die Auflageebene (11) und die Kompressionsfläche (12, 27) in der Kompressionsstellung (B) parallel zueinander ausgerichtet sind.

5. Röntgendiagnostikgerät nach einem der Ansprüche 1 bis 4,
**dadurch gekennzeichnet,**
daß an der patientennahen Stirnseite (26) der Kompressionsplatte (18) eine Andruckfläche (21) eines Abweiselementes (19) um eine zur Stirnseite (26) der Kompressionsplatte (18) parallele Achse (20) schwenkbar gelagert ist und daß eine Anlenkung (22, 23) vorgesehen ist, durch die die Andruckfläche (21) vor der Kompression des Aufnahmeobjektes einen spitzen Winkel zur Kompressionsfläche (27) einnimmt und durch die sich dieser Winkel bei Kompression des Aufnahmeobjektes vergrößert.

## Claims

1. X-ray diagnostic device for mammography exposures with a seating plate (2) for an object to be exposed and with a compression plate (8,18) able to be adjusted perpendicularly relative to the seating plate (2) by way of holding means (4,7,9), wherein the compression surface (12,27) of the compression plate (8,18) and the seating plane (11) of the seating plate (2) in a position A form an acute angle with each other, so that the space between the seating plane (11) and the compression surface (12,27) tapers towards the side of the patient and wherein the end side (13,26) of the compression plate (8,18) is rounded off.

2. X-ray diagnostic device according to claim 1, characterised in that the compression plate (8,18) is mounted by way of the holding means (7,9) such that the angle which the compression surface (12,27) forms with the seating plane (11) is reduced when there is compression of the object to be exposed.

3. X-ray diagnostic device according to claim 1 or 2, characterised in that the seating plane (11) is aligned perpendicularly to a holder (4) and in that the angle which the seating plane (11) and the compression surface (12,27) form with each other is reduced against the force of a spring element (10) when there is compression of the object to be exposed.

4. X-ray diagnostic device according to one of claims 1 to 3, characterised in that the seating plane (11) and the compression surface (12,27) are aligned parallel to one another in the compression position (B).

5. X-ray diagnostic device according to one of claims 1 to 4, characterised in that on the end side (26) of the compression plate (18), close to the patient, there is mounted a pressing surface (21) of a deflecting element (19) able to swivel about an axis (20) parallel to the end side (26) of the compression plate (18) and in that a hinge (22,23) is provided, by means of which hinge the pressing surface (21) forms an acute angle with the compression surface (27) before compression of the object to be exposed and by means of which hinge this angle is enlarged when there is compression of the object to be exposed.

## Revendications

1. Appareil de radiodiagnostic pour la réalisation de mammographies, comportant une plaque d'appui (2) pour un objet d'examen et une plaque de compression (8, 18) déplaçable perpendiculairement à la plaque d'appui (2), par l'intermédiaire de moyens de fixation (4, 7, 9),
la surface de compression (12, 27) de la plaque de compression (8, 18) et le plan d'appui (11) de la plaque d'appui (2) dans une position A, faisant entre eux un angle aigu de sorte que l'espace entre le plan d'appui (11) et la surface de compression (12, 27) se rétrécit en direction du patient, et
la face frontale (13, 26) de la plaque de compression (8, 18) est arrondie.

2. Appareil de radiodiagnostic suivant la revendication 1, caractérisé par le fait que la plaque de compression (8, 18) est supportée par les moyens de fixation (7, 9) de telle sorte que l'angle de la surface de compression (12, 27) avec le plan d'appui (11) diminue lors de la compression de l'objet de prise de vue.

3. Appareil de radiodiagnostic suivant la revendication 1 ou 2, caractérisé par le fait
que le plan d'appui (11) est perpendiculaire à un support (4), et
que l'angle, que font entre eux le plan d'appui (11) et la surface de compression (12, 27), diminue lors de la compression de l'objet de prise de vue, à l'encontre de la force d'un élément de ressort (10).

4. Appareil de radiodiagnostic suivant l'une des revendications 1 à 3, caractérisé par le fait que le plan d'appui (11) et la surface de compression (12, 27) dans la position de compression (B), sont parallèles.

5. Appareil de radiodiagnostic suivant l'une des revendications 1 à 4, caractérisé par le fait qu'une surface de serrage (21) d'un élément formant butoir (19) est montée sur la face frontale (26), proche du patient, de la plaque de compression (18), de manière à pouvoir pivoter autour d'un axe (20) parallèle à la face frontale (26) de la plaque de compression (18), et qu'il est prévu un système articulé (22, 23), grâce auquel la surface de serrage (21) est amenée, avant la compression de l'objet de prise de vue, dans une position faisant un angle aigu avec la surface de compression (27) et à l'aide duquel cet angle est augmenté lors de la compression de l'objet de prise de vue.
